# EUROPEAN PATENT APPLICATION

(11) **EP 3 620 439 A1**
(43) Date of publication of application: **11.03.2020**
(21) Application number: 19194441.2
(22) Date of filing: 29.08.2019
(51) Int. Cl.: C02F 11/00, C02F 1/66, A01C 23/00, C05F 17/40

(54) **A GAS EMISSION REDUCING SYSTEM AND METHOD FOR REDUCING GREEN HOUSE GASES AND/OR AMMONIA EMISSIONS FROM SLURRY STORED IN ONE OR MORE SLURRY STORAGE TANKS**

(30) Priority: 29.08.2018 DK PA201870555
(71) Applicant: Toft, Morten, 6600 Vejen (DK)
(72) Inventor: Toft, Morten, 6600 Vejen (DK)
(74) Representative: Olesen, Birthe Bjerregaard

(57) **Abstract**

A system and a method for reducing emission of greenhouse gasses is disclosed, in particular methane, laughing gas and/or nitrogen oxides, and/or ammonia from slurry stored in one or more slurry storage tanks. The method comprises continuously maintaining slurry stored in a slurry storage tank under acidic conditions.The method includes the steps of
A: monitoring pH in the slurry present in the slurry storage tank by means of one or more pH sensors arranged in contact with the slurry in the slurry storage,
B: checking if the detected pH exceeds above an upper threshold, such as an upper threshold set at pH=7,
C: activate acid addition when the monitored pH exceeds the upper threshold,
D: while stirring, adding acid until pH in the slurry is adjusted to within a range between the upper threshold and a lower threshold, such as between pH=2 and pH=7, in particular between pH=5 to pH=7, or more preferred pH=5 and pH=6, and - repeating steps C-D when the detected pH of step A exceeds the upper threshold.

The system disclosed is adapted for automated control of the acid addition and may simplify slurry handling and/or management as well as reduce the farmer's costs for slurry handling/management and/or acid handling or storage equipment for the farmer.

## Description

### Field of the Invention

The present invention relates to a method for reducing emission of greenhouse gasses, in particular methane, laughing gas and/or nitrogen oxides, and/or ammonia from slurry stored in one or more slurry storage tanks by addition of acid.

The present invention also relates to a gas emission reducing system for reducing emission of greenhouse gasses, in particular methane, laughing gas and/or nitrogen oxides, and/or ammonia from slurry stored in one or more slurry storage tanks,

### Background of the Invention

95% of the ammonia emission in Denmark comes from agriculture. Ammonia emission is unwanted because of the eutrophication damage to nitrogen sensitive areas and the known effects of ammonia in the Particulate matter PM 2.5 air pollution, where ammonia is responsible for as much as 40% of the PM 2.5 air pollution.

The vast majority of these emissions are coming from slurry/manure, i.e. a mixture of animal urine and dung, either during storage in large storage tanks or when/after the slurry is applied in the field for fertilizing purposes.

Acidification is growing in recognition for its ability to control emissions of ammonia (NH₃). Science papers reveal a large amount of articles describing the effect of in house, storage and in field acidification techniques. These have all been developed in Denmark, as a result of political pressure on the market to reduce ammonia emission.

The chemical reaction behind acidification is simple: NH₃ (ammonia)+ H₂SO₄ (sulphuric acid) = 2NH₄ (ammonium) + SO₄ (Sulphate).

The ammonia gas is through equilibrium transformed into ammonium salt and this reaction reduces emissions significantly. The sulphate is a Sulphur fertilizer.

There are currently 3 different versions of the technology
- Barn acidification
- Short term Storage acidification
- In Field acidification

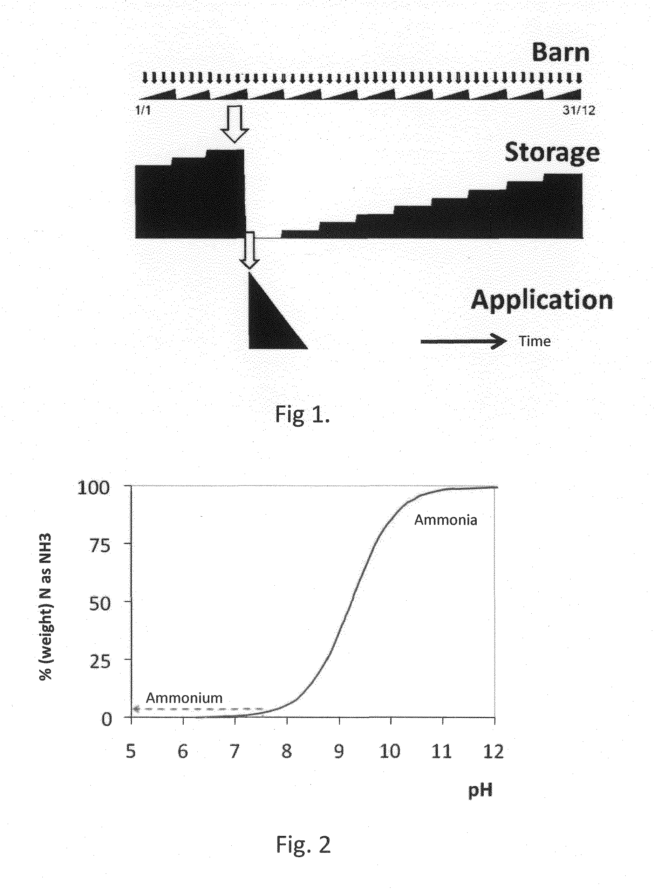

All are described in the science paper Acidification of animal slurry a review
David Fangueiro a, *, Maibritt Hjorth b, Fabrizio Gioelli c Journal of Environmental Management 2014 Elsevier Ltd, 11 pages. Barn acidification typically circulates the slurry in channels while continuously adding acid and ensuring a low pH and thus low ammonia emission inside the barn/stables. This is illustrated in the upper section shown in fig. 1 (marked "Barn"). An example of Barn acidification is disclosed in e.g. US 2004/0040516 A1). The arrows shown in fig. 1 represent acid addition to the slurry while the black blocks represent the lpH level and the related level of ammonia emission relative over time, which is from March to August in fig. 1.

In Denmark, storage acidified slurry is exempt from a legal requirement of slurry cover as there are minimal emission and no crust formation.

Short term storage acidification typically adds acid to the slurry storage once to ensure pH is low enough to keep ammonia emission at a minimum during a long period. A stationary technology to acidify the slurry as it enters the slurry storage tank is already used in Denmark. A typical acid consumption is 4-5 liters pr. m³, so for a normal 2000 m³ storage tank, 8-10 m³ acid is used. This may be considerably less in regions where slurry "flush technology" - a slurry technology where the slurry is diluted with water and recirculated to clean out new manure facieses - is used and where the slurry is often diluted with water. The acid is normally transported to farm by use of a semi-trailer tanker build for ADR (dangerous goods) transport. Then, the (sulphuric) acid is transferred to an acid storage facility at the farm. From there, the farmer may carry our dosing of acid to the slurry, e.g. by addition as the slurry enters the slurry storage tank. This requires that the farmer invests in acid handling equipment and is trained specially in handling the acid.

The amount of acid added in short term slurry acidification usually ensures that pH is lowered to below 6 to or even below 5. During storage of the slurry, and because more slurry is gradually added over the storage period, pH in the slurry in the storage gradually increases. This increases the concentration of ammonia in the slurry and thus increases the risk of ammonia evaporation and thus increased emissions of ammonia to the atmosphere. This is also illustrated in fig 1, middle section (marked "storage").

The distribution of acids to existing slurry storage tanks conventionally is done by a tanker semi-trailer truck with acid carrying ability but the mixing is a separate operation. The tanker typically meets up with a tractor with a slurry mixer at the slurry storage tank. The mixer needs up to 200 hp and is driven from the PTO via the tractor engine. The mixer ensures thorough mixing of the slurry with the acid. The mixer system may e.g. be either a propeller or pump system. The truck and the mixer on the tractor are coupled through hoses and the sulphuric acid is delivered to the slurry through either a pump or a venturi ejector system in the slurry pump or slurry mixer. The venturi ejector creates a vacuum and sucks the acid directly from the semi-trailer tanker via the connected hoses.

Mixing acid into slurry in a slurry storage facility is a challenge because concentrated sulphuric acid has a weight ratio of 1:1.84 to water. If concentrated sulphuric acid is not mixed properly into the slurry, the weight difference will serve to keep the acid concentrated in "pockets" in the body of slurry stored in the slurry storage tank. This may create an explosive environment through heat derived from the dissolvent contact with liquid slurry. Thus, the concentrated sulphuric acid must be thoroughly mixed into the slurry under extreme turbulence from a high volume of slurry. The process of mixing acids into slurry must be done with caution as it releases lots of hydrogen sulfide. Also bicarbonate in the slurry is released as CO₂, which creates foaming. Normally the foaming is gone within 4-24 hours. After acidification, the slurry is far more homogeneous with much less sedimentation between liquid and dry matter, making it easier to mix before application. In areas where drag hose systems for slurry application is used, this is a significant improvement of the pumping ability.

In "in field acidification", the slurry is acidified during application at the field, e.g. by adding sulphuric acid to the slurry in the slurry tanker or by adding acid to the slurry when transferring to being as disclosed in EP 2272316 B1 or EP 2272315 B1. In field acidification ensures a fast and effective reduction of emission of ammonia. Shortly after spreading the acidified manure in the field ammonium is absorbed into the soil and is readily available to the crops, whereby any further ammonia emission is eliminated as also illustrated in fig 1, lower section (marked "application").

What is less known is the acidification technology's ability to also reduce other greenhouse gasses (GHG) like methane (CH₄), nitrous oxide, also known as dinitrogen oxide or laughter gas (N₂O), and other nitrogen oxide pollutants (NOx). This is documented in science papers for both pig and cattle slurry - "Methanogenic community changes and emissions of methane and other gasses, during storage of acidified and untreated slurry" S.O. Petersen1, O. Hojberg, M. Poulsen, C. Schwab and J. Eriksen Journal of Applied Microbiology ISSN 1364-5072, 2014 - 13 pages, and "Effects of cattle slurry acidification and ammonia and methane evolution during storage", Author: Petersen, Søren O; Andersen, Astrid J; Eriksen, Jørgen Publication info: Journal of Environmental Quality ; Madison Vol. 41, Iss. 1, (Jan/Feb 2012): 88-94.

The above mentioned known methods of slurry acidification also have huge differences in impact / effect on different emission types:

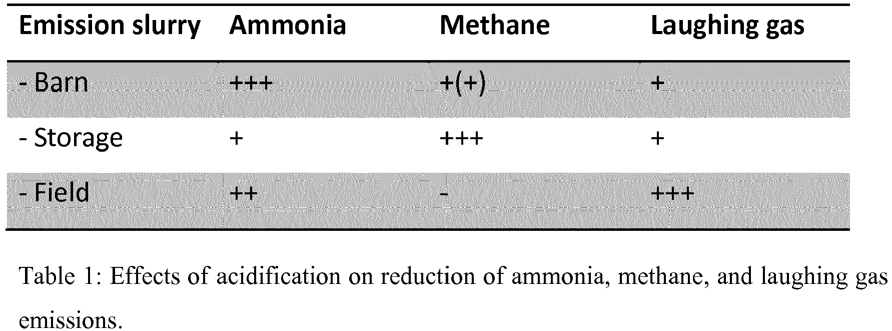

As can be seen from table 1 above, only barn acidification is effective against both ammonia, methane and laughing gas emissions. The storage acidification technology in Denmark is used shortly before the slurry is applied to fields and thus has limited effect on GHG emissions from the slurry storage.

The largest volume of GHG gas from slurry is methane gas. According to Global methane initiative, methane gas from manure management constitutes 3% of the total world methane emission.

In practice, emissions of ammonia and GHG from slurry storages have been reduced by regulations that make covers on slurry storage tanks compulsory. This increases the costs for slurry handling for the farmers and may also cause problems during handling of slurry.

Thus, the reduction of the above mentioned GHG's represents a possibility of obtaining carbon credits. This is at present not worth the effort for each individual farmer to apply for obtaining carbon credits when reducing emission of other GHG's than Carbon dioxide from the farmer's slurry storages. The effort will simply be too time consuming for each farmer in relation to income obtained when selling these carbon credits, since these carbon credits do not represent a large value for the individual farmer. For example, a 2,000 m3 slurry tank from 200 dairy cows in Denmark, would hold a current value of 50 DKK pr. ton CO2 - 200 cows x 17 kg = 3.4 ton CO2 = 170 DKK. Even with growing herd sizes, this is not likely to be worth the effort of an individual farmer to apply for and obtain a carbon credit and find a buyer for it.

The problem of documenting methane emission from manure management facilities is also diffuse, meaning that it is spread out over +100,000 slurry manure storage tanks. These may be located under animal housing or under free air.

The IPCC (UN climate panel) has specified the aim of reducing methane emission from slurry storage according to regional climate conditions. In Europe, the methane emission is from 17 kg to 38 kg pr. dairy cow depending on climate conditions. That means it is not necessary to quantify the emission to apply for a carbon credit, but it is necessary to document the reduction of emissions in % of the established IPCC emission level in order to obtain a carbon credit.

"Emissions trading", or cap and trade, is a market-based approach to provide an economical incentive for reducing the overall emission of carbon dioxide and other GHG's.

A carbon credit can be sold and/or bought, and thus allow for trading the possible emission of Carbon dioxide and other GHG's in an amount corresponding to each carbon credit. Thus, the carbon credit system is a market-based approach to controlling pollution by providing economic incentives for achieving reductions in the emissions of pollutants. In contrast to command-and-control environmental regulations such as best available technology (BAT) standards and government subsidies, cap and trade (CAT) programs are a type of flexible environmental regulation that allows organizations to decide how best to meet policy targets. Various countries, states and groups of companies have adopted such trading systems, notably for mitigating climate change. A central authority (usually a governmental body) allocates or sells a limited number of permits to discharge specific quantities of a specific pollutant per time period. Polluters are required to hold permits (counted in carbon credits) in amount equal to their emissions. Polluters that want to increase their emissions must buy permits from others willing to sell them. Financial derivatives of permits can also be traded on secondary markets.

In theory, polluters who can reduce emissions most cheaply will do so, achieving the emission reduction at the lowest cost to society. Cap and trade of carbon credits is meant to provide the private sector with the flexibility required to reduce emissions while stimulating technological innovation and economic growth. There are active trading programs in several air pollutants. For greenhouse gases, which cause climate change, permit units are often called carbon credits. The largest greenhouse gases trading program is the European Union Emission Trading Scheme, which trades primarily in European Union Allowances (EUAs), the Californian scheme trades in California Carbon Allowances, the New Zealand scheme in New Zealand Units and the Australian scheme in Australian Units. The United States has a national market to reduce acid rain and several regional markets in nitrogen oxides.

At present no systems or methods are available on the market which enable the documentation of reduction of emission of GHG's from manure storage facilities at the individual farm.

Thus, there exists a need for alternative and effective methods for treating slurry and effectively reducing emissions of ammonia as well as GHG, in particular methane and/or laughing gas and/or other nitrogen oxide gasses.

In the present application, the use of sulphuric acid is preferred. Other acids may be used, such as phosphoric acid (e.g. if increase of Phosphorus content in manure is desired) and/or organic acids, e.g. formic acid, acetic acid, citric acid and/or other nontoxic organic acids.

### Object of the Invention

The object of the present invention is to provide a new hybrid version of acidification of slurry that provides a long term storage acidification that maintains a low pH in the storage tank throughout the storage period.

The object of the present invention is also to effectively reduce emissions of ammonia as well as GHG, in particular methane and/or laughing gas and/or other nitrogen oxide gasses from slurry.

The object of the present invention is also to effectively reduce the overall investments in farm equipment, especially for acidification, transport and/or storage of slurry.

The object of the present invention is also to provide a system and a method that effectively ensures long term slurry acidification during slurry storage in slurry storage tanks.

### Description of the Invention

These objects, which are obtained and the above mentioned drawbacks, are overcome by the present invention that provides a method for reducing emission of greenhouse gasses, in particular methane, laughing gas and/or nitrogen oxides, and/or ammonia from slurry stored in one or more slurry storage tanks, which method comprises continuously maintaining slurry stored in a slurry storage tank under acidic conditions, i.e. pH below 7. The method includes the steps of
A: monitoring pH in the slurry present in the slurry storage tank by means of one or more pH sensors arranged in contact with the slurry in the slurry storage,
B: checking if the detected pH exceeds above an upper threshold, such as an upper threshold set at pH=7,
C: activate acid addition when the monitored pH exceeds the upper threshold,
D: while stirring, adding acid until pH in the slurry is adjusted to within a range between the upper threshold and a lower threshold, such as between pH=2 and pH=7, in particular between pH=5 to pH=7, or more preferred pH=5 and pH=6, and - repeating steps C-D when the detected pH of step A exceeds the upper threshold.

Hereby a new hybrid version of slurry storage acidification is suggested. The method can be defined as long term storage acidification that maintains a low pH in the storage tank throughout the storage period. Thereby, the slurry stored in the slurry storage tank is at all times kept at a pH that is low enough to ensure that ammonia is present in the liquid slurry as ammonium. This reduces ammonia emissions significantly or may even eliminate ammonia emissions from the slurry present in the slurry storage.

A higher utilization rate of nitrogen of 30% to 40% in slurry (ammonia converted to ammonium) is also a contribution to less GHG through less need for production and transport of mineral fertilizers and thus a significant contribution to reduction in GHG. The use of acidification technology against GHG's is possible if the slurry is acidified as it enters the slurry storage tank or if the slurry in the tank is repeatedly acidified while in storage.

The methane emissions from manure management originates from methanogenic bacteria activity in the storage area of animal and digested slurry. Acidification is a very effective technology to stop methanogenic bacteria activity. When the slurry pH is lowered below pH 5.5, the methanogenic bacteria cannot thrive in the low pH environment and is virtually eliminated from the slurry and there is no methane gas emission - 84% to 90% reduction ref. above mentioned papers.

The pH in the slurry is not stable after acidification. It is known from the literature that slurry acidified to below pH 5.5, will remain below pH 6.0 from 3 weeks up to 3 months.

In addition, new variable volumes of slurry are added to the slurry storage facility throughout the season, either continuously or in batches when the manure is cleared from the barn. If storage acidification is to be effective towards ammonia and/or multiple GHG's, the slurry in the storage must be stored at pH below 6, preferably at around pH 5.5. It will be equally effective if the pH is lowered to pH 5.0 and allowed to increase to pH 6.5 and then lowered again to pH 5.0. Average pH over time is preferably held at around pH 5.5 - 6.0. To achieve this, repeated acidification of the slurry over a series of additions during the season must take place. All slurries/slurry storage facilities are individual and will require an individual timing and amount of acid. This requires a continued pH monitoring and acidification process where each storage facility has its own pH monitoring. That means that monitoring of the pH is an essential parameter to control the emissions and to document the GHG emission reduction or the environmental effect.

The slurry pH increase is monitored via one or more pH sensors arranged in the slurry storage facility. The sensors may be permanently mounted in the slurry storage, or may e.g. be arranged in a flowing manner in the liquid slurry stored in the slurry storage. Alternatively the pH sensor may be a mobile unit, e.g. hand held units or a pH sensor arranged on a crane arm of an acid tanker truck, the latter being described further below.

The pH sensor data are transferred to a control system, e.g. via wired or wireless technology, such as convention wireless computer networking technologies and/or telecommunication means. The control system monitors pH of the slurry present in the slurry storage and checks if the detected pH exceeds above an upper threshold, such as an upper threshold set at pH=7 or preferably pH=6.

If the upper threshold is exceeded, the control system activates acid addition, e.g. by automatically booking an acid tanker truck to visit the relevant slurry storage, or by creating an alarm that enables the farmer or a central service provider, in the following called a hauler, to request acid delivery to the relevant slurry storage where the alarm is detected.

The acid is added to the slurry in the storage while stirring until pH in the slurry is adjusted to within a range between the upper threshold and a lower threshold, such as between pH=2 and pH=7, in particular between pH=5 and pH=6.

These steps C and D are repeated each time the detected pH of step A exceeds the upper threshold.

The pH sensor(s) are preferably connected to the controller using on-line cloud technology and/or via conventional wireless or wired computer networks. Through this data chain, the service provider/hauler is prompted to return to the facility for delivery of acid to the slurry storage when the pH increases over the threshold, normally set at a pH 6.0 - 6.5, which is considered the upper level of pH for an acceptable level of emission reduction. Upon return of the acid tanker truck the pH is again decreased.

The continued acidification maintains the slurry in the slurry storage at low pH and the emissions of ammonia and/or GHG's is thus reduced. Thereby, the farmer may no longer need to invest in covers for the slurry storages. Other advantages are discussed further below.

Preferably, the acid is added to the slurry storage directly from a tanker trailer, and where a crane arm arranged on the tanker trailer adds the acid to the slurry while also performing extensive stirring/mixing the slurry in the slurry storage by means of pumping means or a mixer arranged on the crane arm.

Hereby is obtained that the farmer no longer needs to use his tractor and attached mixing tools when adding acid to the slurry in the slurry tank. The farmer may thus reduce investments in tools specifically for handling acids, mixing and/or addition of acid to the slurry storage(s) on his or her farm. In addition, the farmer no longer needs to be trained in handling dangerous goods and strong acids, in particular when handling concentrated sulphuric acid. The truck driver that drives the acid tanker truck is trained and can thus handle the entire procedure of slurry acidification, in particular when a fully automated and/or monitored procedure according to the present invention is provided.

When a fully automated system is used, as outlined further below, the stored data from the control system may also be extracted to a report which the farmer can use as documentation of e.g. safe storage of the slurry and/or fertilizer value of the slurry in the slurry storage. The report may e.g. be used to inform the authorities at regular intervals or when required by the authorities.

Further sensor means may be provided to monitor the concentration of P, N, methane, and/or other components in the slurry. These sensor means are preferably arranged in the slurry storage or preferably acid tanker, such as on the crane arm or in the crane arm's slurry pump or slurry mixer or the further sensor means are arranged on the tanker and connected to the slurry flow passages by piping and/or hoses.

Hereby is obtained that the concentration of fertilizer components in the slurry in the slurry storage can be tracked and recorded. This ensures that the farmer may dose the amount of slurry to each area in the field more precisely and/more individually depending on soil type and/or conditions or e.g. according to specific fertilizer needs of the crops that are to be grown in the field where the fertilizer from the relevant slurry storage is spread.

Preferably, pH sensor values and/or values from monitoring concentration of P, N, methane and/or other components are then transferred to a database, and where the database contains identification tags for one or more slurry storage tanks, and stores at least the pH values detected in the slurry storage tanks or each of the tagged slurry storage tanks, and optionally also storing one or more values for the concentration of P, N, methane and/or other components.

Hereby is obtained a method that can be fully automated, because the sensors data can be used for automation of the slurry ordering and addition procedure. Thereby, the farmer no longer needs investing in e.g. slurry storage facilities or slurry handling means. The data stored in the database may also be used for documentation purposes as already mentioned above.

An event is preferably created when pH in a slurry storage exceeds the upper threshold, and where said event activates assigning a truck with the acid tanker trailer to access the slurry storage tank for adjusting pH by transfer of acid to the slurry storage tank.

Hereby is obtained that the fully automated slurry addition control system keeps track of the amount of acid added to the slurry storage tank or to each slurry of the slurry storage tanks that are connected to the control system. This also enables the hauler to keep tracks of his own stock and/or to buy more acid whenever necessary. The automated system may assist the hauler in debiting the correct amount of acid delivered to each farmer.

Thus, this event may actuate an automated booking of an acid tank truck to visit the relevant slurry storage. If the acid tanks are provided with positioning system, a nearby acid tanker truck may be assigned to visit the relevant slurry storage. Alternatively, the event may create an alarm, e.g. in a text message on a mobile phone, or by sound/visual alarm that informs the farmer or the hauler to manually initiate acid addition to the relevant slurry storage. The alarm may also be induced in a local computer unit, e.g. a laptop, a tablet or a mobile phone unit with a specially adapted programming that enables alarming the farmer or hauler.

A time stamp is preferably created when acid is added to the tagged slurry storage and where said time stamps are transferred to and stored in the database for each of the tagged slurry storage tanks. This enables precise historical data present in the database. These data may also be used for documentation purposes and/or billing facilities e.g. by extraction of historical data stored in relation to the relevant tagged slurry storage tank.

The above mentioned objects are also achieved by means of a gas emission reducing system for reducing emission of greenhouse gasses, in particular methane, laughing gas and/or nitrogen oxides, and/or ammonia from slurry stored in one or more slurry storage tanks. The system comprises a fleet of one or more acid tanker trucks, such as semitrailer tank trucks that are authorized for transport of sulphuric acid and/or other strong organic or inorganic acids. Each of said one or more acid tanker truck further comprises a crane arm with acid addition means and with mixing and/or pumping means for mixing or stirring slurry in a slurry storage tank and/or for pumping said slurry. Said system further comprises one or more pH sensors arranged to detect pH of the slurry in the one or more slurry storage tanks. Said system further comprises a central control unit comprising, or in communication with, a database, and/or said acid tank trucks and at the at least one pH sensors arranged at each of the tagged slurry storage tanks. Said database comprises identification tags for each of the one or more slurry storage tanks, and where the database further comprise timestamps for each visit from an acid tank truck to each of the tagged slurry storage tanks and pH data received from the at least one pH sensors of each of the tagged slurry storages.

Hereby is obtained that with such a system, investment in equipment for mixing of slurry or handling acid handling the farm is avoided. The system is based on a centralized control system, where many farmers' slurry storage tanks can be monitored and/or serviced with addition of acid. Thus, the system may be arranged so that the farm simply pays a service/subscription fee to the service provider/hauler operator. This is also a lot safer than to have a storage facility for (sulphuric) acid on the farm, which is operated by the farmer. Maintenance of the equipment by the farm is also eliminated.

A fleet of one or more acid tanker trucks, such as semitrailer tank trucks that are authorized for transport of sulphuric acid and/or other strong organic or inorganic acids are assigned to the system. The assigned acid tanker trailers are preferably tagged, so the system can keep track of which acid tanker trailer that visits each of the slurry storages and when. The tags are registered in the central database that is described further below.

The service provider/hauler may visit a significant amount of different slurry storage facilities during a day. The system may keep tracks of when the acid trailers visit a slurry storage, e.g. by registering GPS data for each of the trucks. In Holland, all trucks transporting slurry must by law have a GPS. Further, in Holland, all trucks transporting slurry must also by law be able to measure each load for the macro NPK nutrients in the slurry. This is e.g. done by using a NIR (Near Infra Read) sensor systems see further below.

The distribution of acids is classified as dangerous goods and is subject to the ADR convention. This has a lot of consequences for the design of the tanker, where special breaks and materials are required, yearly inspections, ADR tanker license for the driver, delivery journal, data sheets of chemicals carried, markers on the tanker, fire extinguisher, and various safety equipment on board the vehicle, and finally, the driver must wear protective suit when delivering the acid. All of this may be a problem to a farm operator, but is not a problem to a chemical distribution company/hauler that is to offer the addition of acid to slurry as a service to the farmer.

With this manner of distributing the acid, a storage facility on farm site is made redundant and the farm operators do not have to be certified/trained to handle the acid. Also, acids can be transported from long distances. Even though acid is readily available in Denmark, significant quantities are hauled by truck from Germany or Holland, where it can be obtained at very low prices, because of a considerable surplus as a waste product from industry.

Each of said one or more acid tanker truck further comprises a crane arm with acid addition means and with mixing and/or pumping means for mixing or stirring slurry in a slurry storage tank and/or for pumping said slurry.

The tanker crane arm is preferably equipped with a double function - one for retracting or delivering the slurry to the storage tank and a second for use as a slurry mixing facility with ability to deliver acid and mix it into the slurry to acidify the slurry in a slurry storage tank.

In addition, the crane arm is preferably equipped with hydraulics that allows automatical movement of the arm relative to the slurry storage tank to improve and/or speed up agitation effect of the slurry during addition of the acid. This ensures faster and more effective mixing of the slurry in the slurry storage, and thus reduces the overall time for lowering pH of the slurry in the slurry storage tank to the desired level as already discussed above.

Further, the acid tanker may further comprise a hose adapted for connection between the slurry storage tank or the mixer and the acid tank of the acid tanker that allows fouled air from the acidification process to enter into the acid tank, where the acid tank functions as an air scrubber. This reduces the escape of odours and/or unwanted gasses, e.g hydrogen sulfide, to the surroundings.

A semi-trailer tanker can carry e.g. 17 m³ of (concentrated) sulphuric acid, which will be enough for 4,-5,000 m³ slurry. It will take the tanker 5-7 hours to stir in the acid into the liquid slurry in the slurry storage tank depending on mixing capacity/type. Preferred mixers are venturi ejector type mixers that automatically suck acid into the flowing slurry that passes through the mixer or conventionally used slurry mixers with means for addition of chemicals and/or acid.

A very good mixer for acid injection is described in patent DK 177702 B1, which is incorporated herein by reference. Both a 4,000- and a 40,000 m³ lagoon will require acidification +/- 10 times in a season. This makes the technology suited for mixing slurry and acid in small as well as very large storage facilities. Since the chemical reaction is in equilibrium, there is no risk of lack of effect because of the size of lagoons and the acidification is done repeatedly over a season.

It is very possible to combine the functions of the tractor/mixer with the semi-trailer truck function. This new storage acidification and system includes a special semi-trailer tanker truck designed to ADR requirements to carry acid. It has an integrated crane arm with slurry mixer ability for adding acids to slurry while also stirring the slurry extensively.

Ideally, the semi-trailer tanker could be designed to carry acid, especially 50% to 97% (by weight) sulphuric acid. In addition, the semi-trailer tanker is likewise designed to carry slurry, as well, e.g. when transport of slurry is needed during the season for spreading slurry in the fields. This would enable the service provider of the technology the ability to offer both an acidification technology and a regular slurry transport option for the customer with the same equipment because transport of acid for acid addition and slurry transport do not overlap. Thus, the tanker trailers can be optimized in hourly use compared to the situation where slurry transport and acid transport are delivered by different trucks and/or by different haulers.

The crane arm would thus have to be designed with flexibility to pump slurry to and from the storage facility and into the tanker. Further, an acid mixing ability and the tank layout on the tanker trailer would have to be able to accommodate both acid and slurry according to needed operation.

Slurry transport is a very seasonal business. The slurry tanker truck/acidification combination can be use all year for either slurry acidification or slurry transport, thus greatly improve the operational time for the service provider/hauler. It will enable an efficient method of acidifying the slurry in the slurry storage facility during the filling of the tank. It will enable long distance transport of acids at high speed for slurry acidification without the need for intermediate storage, decreasing the price for acid purchase and acidification.

In USA, a significant amount of acid is transported by rail. A semi tanker acid truck may have the ability to extract the acid directly from the rail carrier, thus avoiding intermediate storage facility, e.g. by suitable pumping means and relevant connectors to the railed tank.

Said system further comprises one or more pH sensors arranged to detect pH of the slurry in the one or more slurry storage tanks, see further description on the position of the pH sensors above. Alternatively, the one or more sensors may be integrated in the acid tank trailer's NIR sensor system as discussed further below.

Said system further comprises a central control unit comprising, or in communication with, a database, and/or said acid tank trucks and at the at least one pH sensors arranged at each of the tagged slurry storage tanks.

The central control unit may be a cloud based system or the central control unit may comprise more servers that are connected to the internet.

Said database comprises identification tags for each of the one or more slurry storage tanks.

The central control unit receives pH sensor data from the sensors arranged in connection with the tagged slurry storage tanks and assigns the data to the relevant tagged slurry storage.

Then, the control unit transfers the data to the database, where at least pH data are stored.

The database preferably further comprises timestamps for each visit from an acid tank truck to each of the tagged slurry storage tanks. The time stamps are e.g. created by the control unit based on GPS tracking data from an acid tank trailer. Alternatively, a time stamp may be created manually when a truck driver scans a sign with a code, e.g. a QR code or a bar code that identifies the relevant slurry tank.

Further, data relating to the amount of slurry delivered may be transferred to the central unit together with the time stamp. Such data may e.g. be provided by detecting the volume of acid that is transferred to the slurry storage or by means of weighing cells that determine the weight of the acid delivered from the weight of the acid tank on the trailer and any remaining acid therein.

A control unit programming comprises pH sensor registration and assigning data from each sensors to the relevant tagged slurry storage tank. The central control unit then transfers these data to the relevant slurry storage tags in the database.

The central control unit checks if the pH in the slurry present in each of the tagged slurry storage tanks exceeds above an upper threshold, such as an upper threshold set at pH=7. Or preferably a pH upper threshold of pH =6 or between 5 and 6.

If the threshold is exceeded, the central control unit activates acid addition when the monitored pH of a certain tagged slurry storage tank exceeds the upper threshold, such as by assigning one of the acid tanks truck to visit the relevant tagged slurry storage tank within a predefined time range. The predefined time period could e.g. be within a week or 1-5 days, such as 2-3 days when a first threshold is exceeded, e.g. pH=6. Optionally, a shorter time period of e.g. 1-48 hours may be assigned if a second upper threshold of pH=7 is exceeded.

This enables a fully automated system, where truck logistics may be integrated into the control unit.

Alternatively, if the threshold is exceeded, the central control unit activates acid addition when the monitored pH of a certain tagged slurry storage tank exceeds the upper threshold, such as by creating an alarm. The alarm may be audible and/or visible and is given from the central unit to prompt the hauler to manually assign an acid truck to visit the relevant slurry storage with PH sensor data alarm.

Alternatively, or in addition, the farmer may receive an alarm and/or a notification, e.g. in a text message, and/or in a local monitoring unit program, e.g. provided as an application in a farmer's computer, or another portable unit, e.g. a tablet or a mobile phone device. The farmer can then manually react on the alarm/notification by ordering the hauler to deliver acid to the relevant slurry storage tank. See also further below.

The system further comprises further sensor means to monitor the concentration of one or more slurry components in each of the tagged slurry storages, in particular N, P methane and/or ammonia/ammonium, where the further sensor means are arranged on the acid tanker truck, such as in connection with the crane arm's mixing and/or pumping means and/or are arranged at the one or more slurry storage tanks, and where said further sensor means are in communication with said control unit and/or database for storing of concentration data from said further sensor means.

The further sensor means are arranged in contact with the liquid slurry circulating through the acid injection and/or mixing means arranged on the crane arm e.g. by being integrated into the mixer/injection unit. Alternatively, the further sensor means may be arranged separately on the acid tank truck, e.g. in a detachable fitting that is arranged between a hose or piping means that is connected to the slurry tank truck and the acid mixing and/or the slurry mixing means.

It is an advantage if the nutrient data will be part of this new method of acidification of slurry because it enables that the farmer has precise data on the fertilizer content in a certain slurry storage tank. The results of the concentration may be obtained whenever the acid tank visits the relevant slurry storage tank.

The NIR systems for slurry analysis offer a multitude of data such as NPK values, dry matter (TS) and pH. This system may be used when adding acid to the slurry storages. The huge benefit is that the semi-trailer acidification technology will visit a large number of slurry storage tanks. The NIR system can measure an almost unlimited amount of samples at the same cost. This enables a cost sharing between many customers and thus offers a significantly reduced price for nutrient sampling of the slurry. Such a system will thus overcome the use of inaccurate normative figures for nutrients and offer a cost efficient measurement of nutrients to farms.

The nutrient concentration data will comprise a number of discrete data that are collected at each visit by an acid tank trailer truck. These data are stored in the database and together with the relevant tagged slurry storage tank, and any other data related to the relevant tagged slurry storage tank.

The acid tanker truck preferably comprises a data logging means to log one or more data related to a tagged slurry storage, in particular timestamps for a visit to a tagged slurry storage, the amount of acid added to the tagged slurry storage, pH before and/or after addition of acid and/or concentration of further slurry components in particular nutrient data.

This enables logging of data and transfer thereof to the central control unit. The data logger means may be in online communication with the central control unit whereby the detected data may be transferred directly to provide real time information to the hauler's central control unit and/or to the farmer's monitoring unit.

Alternatively, the data logger means may comprise a local data storage where data are stored until the acid tank truck returns to a hauler central or a central/decentral acid loading station. Then the data logger may be connected to a data transfer unit that can transfer the data to the control unit or connected directly to the central unit, e.g. by a wired connection or by wireless connection.

A local monitoring unit comprises monitoring means for monitoring the actual pH and/or concentration data and allows access to the data in the database that are related to one or more predefined tagged slurry storage tanks.

Hereby is obtained that the farmer is able to monitor the slurry in the relevant slurry storage tank or tanks on the relevant farm or farms.

The farmer's local slurry storage monitoring unit may further comprise computing means that can assist the farmer in computing the amount of slurry to spread in a certain field from data related to the soil type and/or condition, crop type etc.

Preferably, each of the sensor means or datalogger units on the acid tanker trucks, and/or the tagged slurry storage tanks and/or the farmer's monitoring unit, are in wireless contact with the control unit and/or the central database, such as via a cloud based communication link and/ or mobile phone connections.

Through this new method and system, it is possible to build a new business model, where the farmer buys GHG reduction/acidification as a service. The service is based on an automatic registration of the pH in the slurry storage facility. A pH sensor in the slurry storage tank is linked to a cloud server that continuously will inform the contractor/farmer about the status of the pH in the storage facility and automatically prompt the contractor to come and lower the pH with the semi-trailer acidification system, in accordance to agreement.

This will relieve the farmer of own investment, give him documentation for his GHG sustainability and earn him an extra income through extra plant available nitrogen and Sulphur. It will also give him valuable information on the amount of nutrients available in the slurry rather than using normative figures and acidification reduces his ammonia emission from 30% to 40%. This constitutes about 1 to 1.5 kg nitrogen pr. m³ and the NIR system is able to inform the customer of this effect and thus quantify the value and the effect of the treatment. The combination of increased efficiency of nitrogen and Sulphur makes the method profitable for the farmer. Economy in acid delivery is greatly dependent on volume of acid transported and volume of slurry treated.

It is expected that a volume from 100,000 m³ slurry treatment will make a very attractive business for a contractor/hauler.

Further, the method may comprise
E: using the documentation of the pH value to obtain a carbon credit corresponding to the emission reduction of methane and/or other greenhouse gases from the one or more slurry storage tanks; and/or
F: gathering one or more carbon credits corresponding to the emission reduction of methane and/or other greenhouse gases from the one or more slurry storage tanks, and optionally, registering any carbon credits obtained in relation to each slurry storage tank in the database.

Further, the system may comprise that data in the database is used for the documentation of the pH value of each individual slurry tank.

Further, this system provides a possibility of providing as documentation for obtain one or more carbon credits relative to a calculated methane emission reduction for each of the individual slurry storage tanks, and wherein a second time stamp is created for each of the carbon credits obtained from each of the one or more slurry storage tanks.

The invention further enables collection of documentation of the diffuse GHG emission from the multiple slurry tanks problem that represents little cap and trade value for an individual farmer, but collectively may be of value.

The problem of Methane emission from manure management is diffuse, meaning that it is spread out over +100.000 slurry manure storage tanks. They may be located under animal housing or under free air.

The IPCC (UN climate panel) has specified the methane emission from slurry storage according to regional climate conditions. In Europe, this is from 17 kg to 38 kg pr. dairy cow depending on climate conditions. That means it is not necessary to quantify the emission to apply for a carbon credit, but it is necessary to document the reduction of emissions in % of the established IPCC emission level. This is possible by using the pH value of the slurry. With the use of sulphuric acid to lower the pH to a level of 6.5 and below, science papers agree on a min. 60% reduction of Methane gas emission and up to 99% reduction. At the same time, this results in a +50% ammonia gas emission.

With such data, it is possible to obtain a carbon credit for the reduction of Methane gas emission (GHG gas emission).

At present, obtaining a carbon credit is too time consuming, and time spent for applying for these carbon credits does not worth the effort since these carbon credits do not represent at large value for the individual farmer. For example, a 2000 m3 slurry tank from 200 dairy cows in Denmark, would hold a current value of 50 DKK pr. ton CO2 - 200 cows x 17 kg = 3.4 ton CO2 = 170 DKK. Even with growing heard sizes, this is not likely to be worth the effort of an individual farmer to apply for and obtain a carbon credit and find a buyer for it.

However, a service provider, that sells acidification as a service to the farmer and also provides the farmer with the documentation of the pH value in the slurry as provided in the central database of the system according to the present invention, may as a obtain the rights for the carbon credit on behalf of the farmers owning each slurry tank tagged in the present system's database. Thereby the service provider can collect data from the database and pool the data into one application for carbon credit(s). Thereby it becomes economically attractable to apply for carbon credits for these diffuse GHG emission reductions and render it profitable to offer the Methane gas emission reduction from slurry management. Thereby, the individual farmer will also have his or her costs for reducing methane emissions from the slurry storage tanks owned by the farmer.

The volume of methane gas emission from slurry is determined by the international climate panel IPCC. In the 2006 guidelines for National Greenhouse Gas Inventories - chapter 10, it is possible to identify the volume of methane gas pr. Animal Unit/ year, barn type and depending on climate zone.

In addition, the California Environmental Protection Agency has created a "Compliance offset protocol Livestock Projects" or capturing and Destroying Methane gas from Manure Management Systems, as a means of issuing cap-n-trade CO2 quotas for reducing methane gas.

From several science papers, among them Journal of Applied Microbiology ISSN 1364-5072 - Methanogenic community changes, and emissions of methane and other gases, during storage of acidified and untreated pig slurry - it is very clear that lowering the pH is a highly effective method of reducing methane gas emission as well as ammonia emission.

As an example of a calculation model of carbon credits based on emission reduction of a greenhouse gas, here Methane in the example, is given below. It is noted that emission reduction of other green house gases may be included in the model if necessary. Methane gas is a greenhouse gas x 24 stronger than CO2.

The above combination creates a new potential income for farmers by allowing their slurry to be acidified. For example, the above mentioned article, mentions that effective acidification of slurry in slurry storage tanks may reduce the methane emission by up to 90% or more.

The combination represents an opportunity to quantify the methane gas emission and create a system in which the methane reduction is transferred into CO2 credits,. These carbon credits can be obtained and sold, e.g. in a cap-n-trade quota:

### Calculation example:

37 kg methane pr. Animal Unit (AU) (IPCC manure management - California emission level)
Average dairy herd size in California 1,000 AU
1,000 AU x 37 kg methane = 37-ton methane.
90% emission reduction = 33-ton x conversion factor 24 = 792-ton CO2
Current CO2 quota pr. ton = 15 $
792-ton CO2 x 15 $ = 11.880 $ potential income from sale of CO2 carbon credits.

The example is based on that a documentation of pH is available, i.e. that pH is maintained at a pH 5.5-6 or below, for the relevant slurry storage tank(s) used for storing slurry from the animals .

Since the cap-n-trade system of carbon credits (quota) is in the beginning of being effective, the predictions are that the quota price will double before flattening out. Since 2014 it has been on a steady increase. The current 15 $ quota price makes acidification in California profitable and with a future price over 20 $, it will be more profitable to invest in slurry management than to increase efficiency in dairy production.

All emission reduction quota systems must identify a method of documenting the gas emission continuously in order to issue a CO2 quota. This must be documented in a protocol that can be repeated endlessly.

The ability to measure pH in slurry and the use with acidification is a long-time recognized measurement method to control ammonia emission. The combination of pH and correlating it to measuring methane gas emission is not a known practice.

A new protocol to this effect can be drafted and approved by the cap-n-trade authorities.

Using acidification and pH is a most advantageous method as it has a double effect and it can document both methane emission and ammonia emission.

Many slurry tanks - or slurry lagoons - are of such a scale that air measurement systems cannot accurately demonstrate the emission or emission reduction.

As pH is an equilibrium in the slurry liquid, it will be effective in all parts of the slurry storage facility and thus it is very well suited to be used for emission documentation.

In addition, it is an inexpensive continuous measurement principle, which is easily installed and provides effective and simple documentation for emission reduction of methane and/or other green house gases from slurry storage tanks.

It is also well documented that acidification of slurry of pH 6.5 and below, breaks calcium bound phosphorus in about only 2 minutes and makes the phosphorus plant available. This is up to 40% of the total volume of phosphorus in dairy slurry. This increased phosphorus fertilization effect makes slurry able to replace mineral starter fertilizers for maize plants and further increases the farm economy.

One or more additives, such as nitrogen inhibitor, manganese nitrate, iron sulfate and/or other slurry additives, may be injected into the slurry during the mixing process, where acid is mixed into the slurry. The additives may be dosed by pumping means of a venturi ejector as already mentioned in connection with the acid addition.

Thus the system may also further comprise one or more additive tanks on the acid tanker for anhydrous ammonia, nitrogen inhibitor, manganese nitrate, iron sulfate and/or other slurry additives, to be injected to the slurry during the mixing process.

Alternatively, the one or more additives are added to the acid for addition to the slurry together with the acid.

Nitrogen inhibitors are commonly used to inhibit denitrification of ammonia and thus increase the utilization efficiency of ammonia/ammonium as a fertilizer. They can be used with both mineral and organic fertilizers such as slurry. The N- inhibitors are mixed into slurry storage tanks, e.g. together with the acid, and/or injected directly into the slurry flow during slurry field application. Some nitrogen inhibitors on the market are very acidic < pH 2. This creates a problem with the use of the nitrogen inhibitors because extensive safety measures must be respected. Normally between 2 and 6 liters are used pr. ha and it must be dissolved into around 30 m³ slurry. This requires a very efficient slurry mixer system to make sure it is properly dissolved and mixed into the giant volume of liquid associated with storage of slurry or a precision dosage system and a flow monitor onboard a slurry tanker. Because of its low pH nature, the nitrogen inhibitor can be dissolved into the sulphuric acid and be equally effective in its ability to inhibit denitrification. Since long term acidification has not been an issue before, using nitrogen inhibitor incorporated into sulphuric acid has also not been used before. This combination avoids any additional mixing equipment and expense and makes equipment on board a slurry tanker redundant. It offers a continued high efficient mixing ability of the nitrogen inhibitor into the slurry, where it will serve to reduce the leaching of nitrogen into the aquatic environment where it is most unwanted. Instead, it is a contribution to farm economy through a better utilization of nitrogen. It thus reduces the need for mineral fertilizer and has a significant effect on GHG reduction (nitrous oxide reduction).

The methane from manure management originates from methanogenic bacteria activity in the storage area of animal and digested slurry. Acidification is a very effective technology to stop methanogenic bacteria activity. When the slurry pH is lowered below pH 5.5, the methanogenic bacteria cannot thrive in the low pH environment and is virtually eliminated from the slurry and there is no methane gas emission - 84% to 90% reduction ref. above papers. At the lowered pH lactative bacterial activity is predominant.

Digestion of slurry is in Germany (9000 units) and Denmark (target 50% volume of slurry digested by 2030) a growing trend. It is not possible to digest acidified slurry using sulphuric acid as the volume of Sulphur is toxic to methanogenic bacteria and reduces the gas yield when the slurry is digested in biogas plants. Science papers speculate that 20% of biogas slurry can be acidified before entering into the digester reactor, but this is difficult to manage in practice and as a result, operative management of digesters very often refuse to accept acidified slurry. Acidification of digested slurry must thus take place in the storage facility or with in field acidification. The fermentation process aggravates the methanogenic bacteria by increasing the pH, but as the slurry is only allowed 20 days in the reactor, only about 30% of the dry-matter has been mineralized and the methanogenic process continues. Without a gas tight cover and collection of the gas after digestion, the methane emission is significantly increased in comparison with conventional storage of slurry.

During the digestion, a significant amount of hydrogen sulfide is emitted together with the methane. This is often filtered out before the burning of the gas or upgrading of the gas to be used in the grid systems. This often ends in a "grey water" solution and is disposed as a low solution sulphuric acid. The point is, that this is often +20% of the total sulphate in the slurry. In addition, the sulphate in the slurry is immobilized with the methanogenic activity and thus not plant available. Adding sulphuric acid and transforming it to sulphate through acidification is to be regarded as a mineral fertilizer substitute and can be added with the same amount of Sulphur as is required by plant uptake. Plant uptake is variable:

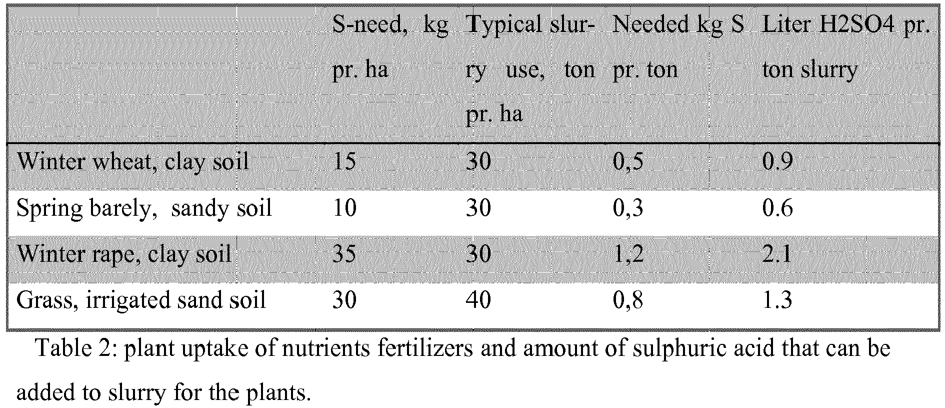

The fertilization value of Sulphur in sulphuric acid can thus be fully offset against sulphate in mineral fertilizers. Especially in digested slurry, it will save a passing of the field having to add additional Sulphur because of the Sulphur emission and immobilization effect of digestion of slurry, creating improved farm economy.

Preferably, anhydrous ammonia may also be added to the slurry while adding acid to the slurry. The anhydrous ammonia is to be added from a separate tank that can be connected to the mixer on the slurry tanker. The ammonia tank may be a stationary tank on the farm or a separate truck with a tanker (semi) trailer loaded with anhydrous ammonia. Patent No. EP 2 514 294 A1 describes a method of adding anhydrous ammonia to slurry on a mobile vehicle and is incorporated herein for reference. The idea is to use fertilizer industry raw material directly at the end consumer level to avoid the cost of having to refine and granulate ammonia into nitrate or semi ammonium/nitrate fertilizer products. Adding anhydrous ammonia to slurry automatically changes the ammonia into ammonium and the addition of acid lowers the pH, stabilizes the ammonium and avoids ammonia emissions and adds sulfate as a fertilizer. This method and system is ideal for adjusting the Nitrogen : Phosphorus (N:P) ratio in the slurry, as there is generally too much P in the slurry. By increasing the N in the N:P ration, a far better utilization of the slurry is achieved and economy and sustainability is improved.

This method and system is known as designer slurry. As the acidification process further breaks the calcium bound phosphorus and makes it plant available, there is a huge potential to increase the utilization rate of the phosphorus in the slurry and to achieve a sustainable use of organic fertilizers while using fertilizer industry raw material (anhydrous ammonia and sulphuric acid) directly in the end user chain. This avoids costly refining methods and reduces the GHG footprint of fertilizer production up to ½ % of world CO₂ emission.

### Description of the Drawing

The present invention will in the following be described in detail with reference to the drawings in which
- Fig.1: shows changes in pH and thus ammonia emission over time - a season - in "in barn" acidification, "in storage" short term acidification and "in field" acidification during spreading of manure/slurry,
- Fig. 2: is a graphic illustration of the weight % of ammonium N that is present as ammonia over pH,
- Fig 3: is a graphical illustration of the computerized system for monitoring pH and controlling addition of acid according to the present invention,
- Fig. 4: shows a rear part of a semitrailer tanker, with a crane arm mounted at the rear end or the tanker, and
- Fig. 5: shows a mixer/pump for mounting at the crane arm.

### Detailed Description of the Invention

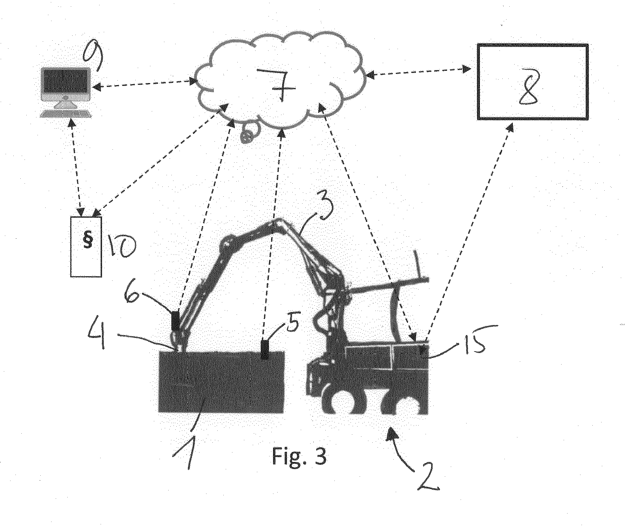

A gas emission reducing system for reducing emission of greenhouse gasses, in particular methane, laughing gas and/or nitrogen oxides, and/or ammonia from slurry stored in one or more slurry storage tanks 1 is illustrated in fig. 3. The system is based on a centralized control system, where many farmers' slurry storage tanks can be monitored and/or serviced with addition of acid. The system comprises a fleet of one or more acid tanker trucks 2, such as semitrailer tank trucks

Each of said one or more acid tanker trucks 2 further comprise a crane arm 3 with acid addition means and with mixing and/or pumping means 5 for mixing or stirring slurry in a slurry storage tank 1 and/or for pumping said slurry.

Said system further comprises one or more pH sensors 5 arranged to detect pH of the slurry in the one or more slurry storage tanks 1.

Said system further comprises a central control unit 8 comprising, or in communication with, a database, and/or said acid tank trucks and at the at least one pH sensors arranged at each of the tagged slurry storage tanks, e.g. via cloud 7 based technology.

Said database comprises identification tags for each of the one or more slurry storage tanks, and where the database further comprises timestamps for each visit from an acid tank truck to each of the tagged slurry storage tanks and pH data received from the at least one pH sensors of each of the tagged slurry storages.

A fleet of one or more acid tanker trucks 2, such as semitrailer tank trucks that are authorized for transport of sulphuric acid and/or other strong organic or inorganic acids are assigned to the system. The assigned acid taker trailers are preferably tagged, so the system can keep track of which acid tanker trailer that visits each of the slurry storages and when. The tags are registered in the central database.

The system may keep tracks of when the acid trailers 2 visits a tagged slurry storage 1, e.g. by registering GPS data (GPS not shown in fig 3) for each of the trucks.

Each of said one or more acid tanker trucks 2 further comprise a crane arm 3 with acid addition means and with mixing and/or pumping means 4 for mixing or stirring slurry in a slurry storage tank and/or for pumping said slurry.

The crane arm would thus have to be designed with flexibility to pump slurry to and from the storage facility and into the tanker. Further, an acid mixing ability and the tank layout on the tanker trailer would have to be able to accommodate both acid and slurry according to needed operation.

Said system further comprises one or more pH sensors 5 arranged to detect pH of the slurry in the one or more slurry storage tanks 1. Alternatively, the one or more sensors may be integrated in the acid tank trailer's nutrient (NIR) sensor system 6 as discussed further below.

Said system further comprises a central control unit 8 comprising, or in communication with, a database (not shown), and/or said acid tank trucks 2 and at the at least one pH sensors 5 arranged at each of the tagged slurry storage tanks 2.

The central control unit 8 may be a cloud 7 based system or the central control unit 8 may comprise more servers that are connected to the internet.

Said database comprises identification tags for each of the one or more slurry storage tanks 1.

The central control unit receives pH sensor data from the sensors 5 arranged in connection with the tagged slurry storage tanks 1 and assigns the data to the relevant tagged slurry storage.

Then, the control unit transfers the data to the database, e.g. in the cloud 7, where at least pH data are stored together with the tags of each slurry tank.

The database preferably further comprises timestamps for each visit from an acid tank truck to each of the tagged slurry storage tanks. The time stamps are e.g. created by the control unit based on GPS tracking data from an acid tank trailer. Alternatively, a time stamp may be created manually when a truck driver scans a sign with a code, e.g. a QR code or a bar code that identifies the relevant slurry tank.

Further, data relating to the amount of slurry delivered may be transferred to the central unit together with the time stamp. Such data may e.g. be provided by detecting the volume of acid that is transferred to the slurry storage or by means of weighing cells that determine the weight of the acid delivered from the weight of the acid tank on the trailer and any remaining acid therein.

A control unit 8 programme comprises pH sensor 5 registration and assigning data from each of the pH sensors 5 to the relevant tagged slurry storage tank 1. The central control unit then transfers these data to the relevant slurry storage tags in the database.

The central control unit 8 checks at regular intervals if the pH in the slurry present in each of the tagged slurry storage tanks 1 exceeds above an upper threshold, such as an upper threshold set at pH=7. Or preferably a pH upper threshold of pH =6 or between 5 and 6.

If the threshold is exceeded, the central control unit 8 activates acid addition when the monitored pH of a certain tagged slurry storage tank 1 exceeds the upper threshold, such as by assigning one of the acid tank trucks to visit the relevant tagged slurry storage tank within a predefined time range. The predefined time period could e.g. be within a week or 1-5 days, such as 2-3 days when a first threshold is exceeded, e.g. pH=6. Optionally, a shorter time period of e.g. 1-48 hours may be assigned if a second upper threshold of pH=7 is exceeded.

This enables a fully automated system, where truck logistics may be integrated into the control unit.

Alternatively, when the threshold is exceeded, the central control unit 8 activates acid addition when the monitored pH of a certain tagged slurry storage tank exceeds the upper threshold, by creating an alarm. The alarm may be audible and/or visible and is given from the central unit to prompt the hauler to manually assign an acid truck to visit the relevant slurry storage with PH sensor data alarm.

Alternatively, or in addition, the farmer may receive an alarm and/or a notification, e.g. in a text message, and/or in a local monitoring unit 9 program, e.g. provided as an application in a farmer's computer, or another portable unit, e.g. a tablet or a mobile phone device. The farmer can then manually react on the alarm/notification by ordering the hauler to deliver acid to the relevant slurry storage tank.

The system further comprises further sensor means 6, such as a NIR sensor as discussed above, to monitor the concentration of one or more slurry components in each of the tagged slurry storages 1, in particular N, P, methane and/or ammonia/ammonium. The further sensor means, i.e. the nutrient sensor 6, is arranged on the acid tanker truck, such as in connection with the crane arm's mixing and/or pumping means 13. Alternatively, the further sensor means 6 are arranged on the tanker and connected to the slurry flow passages by piping and/or hoses (not shown).

Said nutrient sensor 6 is in communication with said control unit 8 and/or database for storing of concentration data from said further sensor means, e.g. by wireless connection or by intermediate storage in a datalogger on the truck as described above.

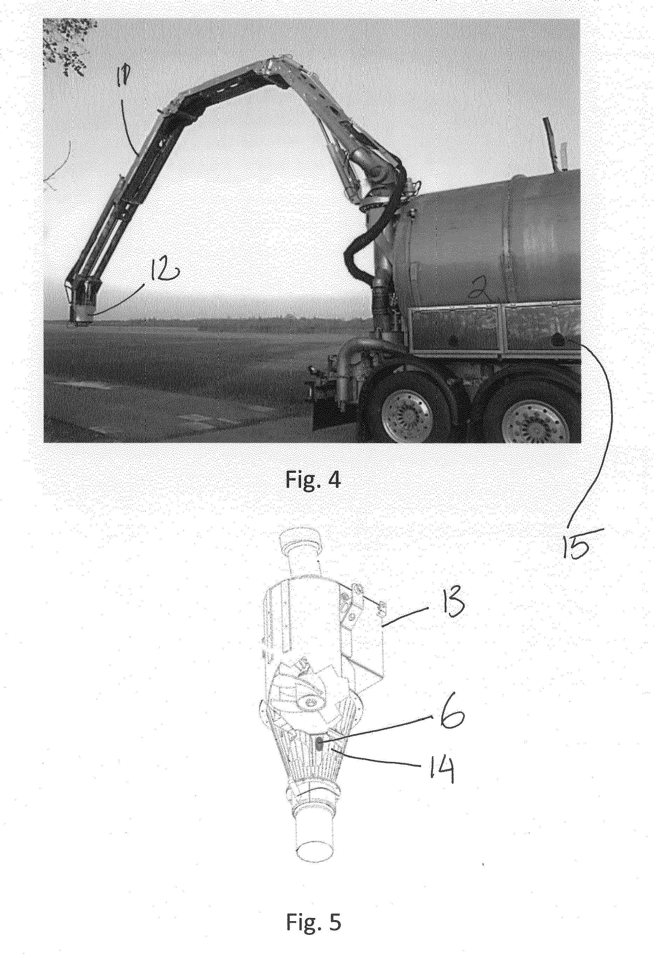

The nutrient sensor 6 is arranged in contact with the liquid slurry circulating through the acid injection and/or mixing means 13 arranged on the crane arm 12. e.g. by being integrated into the mixer/injection unit 13 e.g. as shown in fig. 5, where the nutrient sensor 6 is arranged on or in the slurry flow channel 14 through the mixer head 13. In the slurry flow channel 14, addition of acid is also provided, e.g. by means of an ejector unit (not shown) Alternatively, the further sensor means may be arranged separately on the acid tank truck, e.g. in a detachable fitting (not shown) that is arranged between a hose or piping means that is connected to the slurry tank truck and the acid mixing and/or the slurry mixing means.

The nutrient concentration data will comprise a number of discrete data that are collected at each visit by an acid tank trailer truck. These data are stored in the database and together with the relevant tagged slurry storage tank, and any other data related to the relevant tagged slurry storage tank.

The acid tanker may comprise a data collection and/or transfer unit 15 that is preferably in online communication with the central control unit 8, e.g. via a cloud 7 based link, whereby the detected data may be transferred directly to provide real time information to the hauler's central control unit 8 and/or to the farmer's monitoring unit 9.

The system preferably comprises one or more level sensors (not shown), which are arranged to determine the amount of slurry in the slurry storage tank. The level data are preferably also transmitted to the central control unit and/or stored in the database together with other data related to the tagged slurry storage. This allows the system to determine the actual total volume of slurry present in the slurry storage. Thus, the total slurry volume does not need to be calculated using estimates for evaporation and/or addition of water (e.g. if "flush technology" is applied in the barn.

Alternatively, the level sensor means comprise one or more distance measuring means, positioned on the slurry storage tank, the mixer, the slurry conduit and/or the pump means, wherein the measuring means is adapted for measuring the distance between the top of the slurry storage tank and the slurry surface level, whereby the amount of slurry in the slurry storage tank can be determined.

The farmer's local slurry storage monitoring unit 9 may further comprise computing means that can assist the farmer in computing the amount of slurry to spread in a certain field from data related to the soil type and/or condition, crop type etc. In addition, the farmer's monitoring unit may comprise a reporting module (not shown) that enables the farmer to create reports to control authorities 10 on pH in slurry storage, nutrient concentration and/or when acid addition was performed. In a variant these reports are created automatically and transferred automatically to the control authorities.

Similarly, the hauler's control unit 8 may comprise a second report module for creating reports on acid tanker transports, and any required documentation that the control authorities require for maintaining the hauler's authorisation for transport of dangerous goods.

Preferably, each of the sensor means 5, 6 or datalogger units on the acid tanker trucks 2, and/or the tagged slurry storage tanks 1 and/or the farmer's monitoring unit are in wireless contact with the control unit 8 and/or the central database, such as via a cloud 7 based communication link and/or mobile phone connections as illustrated with the dotted lines.in fig. 3.

### References

- 1.: slurry storage
- 2.: tank trailer - acid tanker
- 3.: crane arm
- 4.: mixer/pump
- 5.: pH sensor
- 6.: nutrient sensor
- 7.: cloud
- 8.: control unit/hauler unit
- 9.: farmer's monitoring unit
- 10.: control authorities
- 11.: crane arm links
- 12.: crane arm end with adaptor for mixer/pump
- 13.: pump/mixer for adaptor
- 14.: slurry flow channel in mixer
- 15.: data collection and/or transfer unit on tanker

## Claims

1. A method for reducing emission of greenhouse gasses, in particular methane, laughing gas and/or nitrogen oxides, and/or ammonia from slurry stored in one or more slurry storage tanks, which method comprises continuously maintaining slurry stored in a slurry storage tank under acidic conditions, and wherein the method includes the steps of
A: monitoring pH in the slurry present in the slurry storage tank by means of one or more pH sensors arranged in contact with the slurry in the slurry storage,
B: checking if the detected pH exceeds above an upper threshold, such as an upper threshold set at pH=7,
C: activate acid addition when the monitored pH exceeds the upper threshold,
D: while stirring, adding acid until pH in the slurry is adjusted to within a range between the upper threshold and a lower threshold, such as between pH=2 and pH=7, in particular between pH=5 to pH=7, or more preferred pH=5 and pH=6, and - repeating steps C-D when the step detected pH of step A exceeds the upper threshold.

2. Method according to claim 1, **characterized in that** the acid is added to the slurry storage directly from a truck tanker trailer, and where a crane arm arranged on the tanker trailer adds the acid to the slurry while also stirring the slurry in the slurry storage by means of pumping means or a mixer arranged on the crane arm.

3. Method according to claim 1 or 2, **characterized in that** further sensor means monitor the concentration of P, N, methane and/or other components in the slurry, wherein the sensors and/or the pH sensor means are arranged in the slurry storage or on the crane arm or in the crane arm's slurry pump or slurry mixer or where the further sensor means are arranged on the tanker and connected to the slurry flow passages by piping and/or hoses.

4. Method according to any of the claims 1 to 3, **characterized in that** pH sensor values and/or values from monitoring concentration of P, N, methane and/or other components are transferred to a database, and where the database contains identification tags for one or more slurry storage tanks, and stores at least the pH values related to the slurry storage tanks or to each of the tagged slurry storage tanks, and optionally also one or more values for the concentration of P, N, methane and/or other components, and wherein a time stamp is created when acid is added to the tagged slurry storage and where said time stamps are transferred to and stored in the database for each of the tagged slurry storage tanks.

5. Method according to any of the claims 1 to 4, **characterized in that** an event is created when pH in a slurry storage exceeds the upper threshold, and where said event activates assigning a truck with the acid tanker trailer to access the slurry storage tank for adjusting pH by transfer of acid to the slurry storage tank.

6. Method according to any of the claims 1 to 5, **characterized in that** anhydrous ammonia is added to the slurry while adding acid to the slurry and/or one or more additives, such as nitrogen inhibitor, manganese nitrate, iron sulfate and/or other slurry additives, are injected into the slurry during the mixing process or the one or more additives are added to the acid for addition to the slurry together with the acid.

7. Method according to any of the claims 1 to 6, **characterized in that** the method further comprises
E. Using the documentation of the pH value to obtain a carbon credit corresponding to the emission reduction of methane and/or other greenhouse gases from the one or more slurry storage tanks, and/or
F. gathering one or more carbon credits corresponding to the emission reduction of methane and/or other greenhouse gases from the one or more slurry storage tanks , and optionally, registering any carbon credits obtained in relation to each slurry storage tank in the database.

8. A gas emission reducing system for reducing emission of greenhouse gasses, in particular methane, laughing gas and/or nitrogen oxides, and/or ammonia from slurry stored in one or more slurry storage tanks, which system comprises
- a fleet of one or more acid tanker trucks, such as semitrailer tank trucks, where each of said one or more acid tanker truck further comprise a crane arm with acid addition means and with mixing and/or pumping means for mixing or stirring slurry in a slurry storage tank and/or for pumping said slurry,
- one or more pH sensors arranged to detect pH of the slurry in the one or more slurry storage tanks, and
- a central control unit comprising, or in communication with, a database, and/or said acid tank trucks and at the at least one pH sensors arranged at each of the tagged slurry storage tanks,
- said database comprises identification tags for each of the one or more slurry storage tanks, and where the database further comprises timestamps for each visit from an acid tank truck to each of the tagged slurry storage tanks and pH data received from the at least one pH sensors of each of the tagged slurry storages.

9. A gas emission reducing system according to claim 8, **characterized in that** the central control unit checks if the pH in the slurry present in each of the tagged slurry storage tanks exceeds above an upper threshold, such as an upper threshold set at pH=7, and activates acid addition when the monitored pH of a certain tagged slurry storage tank exceeds the upper threshold, such as by creating an alarm or by assigning one of the acid tank truck to visit the relevant tagged slurry storage tank within a predefined time range.

10. A gas emission reducing system according to claim 8 or 9, **characterized in that** the system further comprises further sensor means to monitor the concentration of one or more slurry components in each of the tagged slurry storages, in particular N, P, methane and/or ammonia/ammonium, where the further sensor means are arranged on the acid tanker truck, such as in connection with the crane arm's mixing and/or pumping means and/or are arranged at the one or more slurry storage tanks, and where said further sensor means are in communication with said control unit and/or database for storing of concentration data from said further sensor means.

11. A gas emission reducing system according to any of the claims 8-10, **characterized in that** said acid tanker truck comprises a data logger unit to log one or more data related to a tagged slurry storage, in particular timestamps for a visit to a tagged slurry storage, the amount of acid added to the tagged slurry storage, pH before and/or after addition of acid and/or concentration of further slurry components.

12. A gas emission reducing system according to any of the claims 8-11, **characterized in that** a local monitoring unit comprises monitoring means for monitoring the actual pH and/or concentration data and allows access to the data in the database that are related to one or more predefined tagged slurry storage tanks.

13. A gas emission reducing system according to any of the claims 8-12, **characterized in that** the system further comprises one or more additive tanks on the acid tanker for anhydrous ammonia, nitrogen inhibitor, manganese nitrate, iron sulfate and/or other slurry additives, to be injected to the slurry during the mixing process.

14. A gas emission reducing system according to any of the claims 8-13, **characterized in that** the system comprises one or more level sensors being arranged to determine the amount of slurry in the tank.

15. System according to any of the claims 8 to 14, **characterized in that** data in the database is used for the documentation of the pH value of each individual slurry tank and further as documentation for obtain one or more carbon credits relative to a calculated methane emission reduction for each of the individual slurry storage tanks, and wherein a second time stamp is created for each of the carbon credits obtained from each of the one or more slurry storage tanks.
